# EUROPEAN PATENT SPECIFICATION

(11) **EP 2 763 630 B1**
(45) Date of publication and mention of the grant of the patent: **06.04.2016**
(21) Application number: 12733319.3
(22) Date of filing: 21.06.2012
(51) Int. Cl.: A61F 2/915

(54) **MODIFIED SCAFFOLDS FOR PERIPHERAL APPLICATIONS**
MODIFIZIERTE GERÜSTE FÜR PERIPHERE ANWENDUNGEN
TUTEURS MODIFIÉS POUR APPLICATIONS PÉRIPHÉRIQUES

(30) Priority: 03.10.2011 US 201113252121
(43) Date of publication of application: 13.08.2014
(73) Proprietor: Abbott Cardiovascular Systems Inc., Santa Clara, California 95054 (US)
(72) Inventor: PAPP, John E., Temecula, California 92592 (US); TROLLSAS, Mikael, San Jose, California 95124 (US); DAVALIAN, Dariush, San Jose, California 95124 (US); WANG, Yunbing, Sunnyvale, California 94087 (US); HOSSAINY, Syed Faiyaz Ahmed, Hayward, California 94544 (US)
(74) Representative: ZBM Patents - Zea, Barlocci & Markvardsen
(86) International application number: PCT/US2012/043497
(87) International publication number: WO 2013/052184

(56) References cited:
- WO-A1-98/20810
- US-A1- 2002 138 131
- US-A1- 2009 076 584
- US-A1- 2009 182 413
- US-A1- 2010 042 202
- US-A1- 2011 066 225
- US-B1- 6 187 034

## Description

### BACKGROUND OF THE INVENTION

### Field of the Invention

This invention relates to methods of treatment of blood vessels with bioabsorbable polymeric medical devices, in particular, stent scaffolds.

### Description of the State of the Art

This invention relates to radially expandable endoprostheses, that are adapted to be implanted in a bodily lumen. An "endoprosthesis" corresponds to an artificial device that is placed inside the body. A "lumen" refers to a cavity of a tubular organ such as a blood vessel. A stent is an example of such an endoprosthesis. Stents are generally cylindrically shaped devices that function to hold open and sometimes expand a segment of a blood vessel or other anatomical lumen such as urinary tracts and bile ducts. Stents are often used in the treatment of atherosclerotic stenosis in blood vessels. "Stenosis" refers to a narrowing or constriction of a bodily passage or orifice. In such treatments, stents reinforce body vessels and prevent restenosis following angioplasty in the vascular system. "Restenosis" refers to the reoccurrence of stenosis in a blood vessel or heart valve after it has been treated (as by balloon angioplasty, stenting, or valvuloplasty) with apparent success.

Stents are typically composed of scaffolding that includes a pattern or network of interconnecting structural elements or struts, formed from wires, tubes, or sheets of material rolled into a cylindrical shape. This scaffold or scaffolding gets its name because it physically holds open and, if desired, expands the wall of the passageway. Typically, stents are capable of being compressed or crimped onto a catheter so that they can be delivered to and deployed at a treatment

US 2009/0076584 discloses a scaffold wherein the adjacent cylindrical rings comprise link struts connecting all the aligned crests and troughs.

US2009/0076584 discloses a scaffold wherein the adjacent cylindrical rings comprise link struts connecting all the aligned crests and troughs.

Delivery includes inserting the stent through small lumens using a catheter and transporting it to the treatment site. Deployment includes expanding the stent to a larger diameter once it is at the desired location. Mechanical intervention with stents has reduced the rate of acute closure and restenosis as compared to balloon angioplasty.

Stents are used not only for mechanical intervention but also as vehicles for providing biological therapy. Biological therapy uses medicated stents to locally administer a therapeutic substance. The therapeutic substance can also mitigate an adverse biological response to the presence of the stent. A medicated stent may be fabricated by coating the surface of either a metallic or polymeric scaffold with a polymeric carrier that includes an active or bioactive agent or drug. Polymeric scaffolding may also serve as a carrier of an active agent or drug by incorporating a drug through out the scaffolding material.

The stent must be able to satisfy a number of mechanical requirements. The stent must have sufficient radial strength so that it is capable of withstanding the structural loads, namely radial compressive forces, imposed on the stent as it supports the walls of a vessel. This structural load will change as a function of time as the vessel heals, undergoes positive remodeling, or adapts to the presence of the stent. Once expanded, the stent must adequately provide lumen support during a time required for treatment in spite of the various forces that may come to bear on it, including the cyclic loading induced by the beating heart. In addition, the stent must possess sufficient flexibility with a certain resistance to fracture.

Stents implanted in coronary arteries are primarily subjected to radial loads, typically cyclic in nature, which are due to the periodic contraction and expansion of vessels as blood is pumped to and from a beating heart. Stents implanted in peripheral blood vessels, or blood vessels outside the coronary arteries, e.g., iliac, femoral, popliteal, renal and subclavian arteries, however, can undergo significant nonpulsatile forces and must be capable of sustaining both radial forces and crushing or pinching loads. These stent types are implanted in vessels that are closer to the surface of the body. Because these stents are close to the surface of the body, they are particularly vulnerable to crushing or pinching loads, which can partially or completely collapse the stent and thereby block fluid flow in the vessel.

The superficial femoral artery (SFA), in particular, can subject a scaffold to various nonpulsatile forces, such as radial compression, torsion, flexion, and axial extension and compression, which place a high demand on the mechanical performance of implants.

Thus, in addition to high radial strength, stents or scaffolds for peripheral vessels such as the SFA, require a high degree of crush recovery. The term "crush recovery" is used to describe how the scaffold recovers from a pinch or crush load, while the term "crush resistance" is used to describe the force required to cause a permanent deformation of a scaffold.

Stents made from biostable or non-bioerodible materials, such as metals, have become the standard of care for percutaneous coronary intervention (PCI) as well as in peripheral applications, such as the superficial femoral artery (SFA), since such stents have been shown to be capable of preventing early and late recoil and restenosis.

However, in many treatment applications, the presence of a stent in a body is necessary for a limited period of time until its intended function of, for example, maintaining vascular patency and/or drug delivery is accomplished. Moreover, it is believed that biodegradable scaffolds allow for improved healing of the anatomical lumen as compared to metal stents, which may lead to a reduced incidence of late stage thrombosis. In these cases, there is a desire to treat a vessel using a polymer scaffold, in particular a bioerodible polymer scaffold, as opposed to a metal stent, so that the prosthesis's presence in the vessel is for a limited duration. However, there are numerous challenges to overcome when developing a polymer scaffold, particularly in peripheral blood vessels, or blood vessels outside the coronary arteries in which a stent is subjected to both radial forces and nonpulsatile forces.

### SUMMARY OF THE INVENTION

### AS CLAIMED

Various embodiments of the present invention include a scaffold, comprising: two or more radially expandable axial scaffold segments arranged end to end, wherein each segment includes 2 or more cylindrical rings composed of undulating struts, and wherein adjacent cylindrical rings in the same axial segment comprise one or more link struts connecting the adjacent cylindrical rings.

Additional examples include a scaffold delivery system, comprising: a plurality of axial scaffold segments arranged end to end mounted over a cylindrical support, wherein the axial scaffold segments are not connected by link struts.

Other examples include a scaffold, comprising: two or more radially expandable axial scaffold segments arranged end to end, wherein each segment includes 2 or more cylindrical rings composed of undulating struts having crests and troughs, wherein the axial segments are not connected by struts, wherein adjacent cylindrical rings in the same segment are connected by a link struts that have a length less than a ring strut length between a crest and a trough of a ring.

Further examples include a scaffold comprising: a polymeric scaffold composed of a plurality of interconnected struts with gaps in the scaffold between the struts; a plurality of elongate polymeric elements bonded to struts of the scaffold extending across the gaps in the scaffold, wherein axes of the elongate polymeric elements have a component along the axis of the scaffold.

Additional examples nclude a scaffold comprising: a polymeric scaffold composed of a plurality of interconnected struts with gaps in the scaffold between the struts; a tubular polymeric structure bonded to struts of the scaffold extending across and over at least a portion of the gaps in the scaffold.

Further examples include a scaffold, comprising: two for more radially expandable axial scaffold segments arranged end to end, wherein each segment includes 2 or more cylindrical rings composed of undulating struts connected by linking struts, and wherein adjacent cylindrical rings in the same axial segment comprise one or more link struts connecting the adjacent cylindrical rings, wherein adjacent axial segments are connected by flexible links that allow relative axial movement of axial segments.

### BRIEF DESCRIPTION OF THE DRAWINGS

FIG. 1 depicts an exemplary stent scaffold.
FIG. 2 depicts an exemplary scaffold pattern which shows schematically the forces acting on the scaffold.
FIG. 3 depicts a strut section of the pattern depicted in FIGs. 1 and 2.
FIG. 4 depicts a scaffold composed of rings of struts connected by linking struts.
FIG. 5 depicts a scaffold after removal of linking struts showing disconnected axial segments.
FIG. 6A depicts an exemplary axial scaffold segment.
FIG. 6B depicts a close-up view of a portion of the axial segment in FIG. 6A illustrating various features.
FIG. 6C depicts a portion of another exemplary pattern of an axial scaffold segment having fewer than every aligned crest and trough of adjacent rings connected by a short link strut.
FIG. 6D depicts a portion of another exemplary pattern of an axial scaffold segment with keyhole features at the inner surface of the crests and troughs.
FIG. 6E depicts a close-up portion of the exemplary pattern of FIG. 6D with exemplary dimensions for θ, ϕ, H_{c}, W_{c}, Wᵣ, Wₗ, and Lₗ.
FIG. 7 depicts a scaffold composed a plurality of disconnected axial sections from FIG. 6.
FIG. 8 depicts a cross section of disconnected axial segments disposed over a balloon in a deflated configuration.
FIG. 9 depicts a scaffold pattern with axially oriented elongate elements bonded to the scaffold.
FIG. 10 depicts a scaffold pattern with non-axially oriented elongate elements bonded to the scaffold.
FIG. 11 depicts a cross section of a section of the pattern depicted in FIG. 9
FIGs. 12 and 13 depict polymeric elongate elements bonded to a surface of decoupled axial elements.
FIG. 14 depicts an axial projection of a tube of helically wound fiber mesh.
FIG. 15 depicts a fibrous polymer mesh of a fibrous tube.
FIG. 16 depicts an axial projection of a polymer film tube.
FIG. 17 depicts a portion of a scaffold with a fiber mesh tube over a scaffold.
FIG. 18 depicts a portion of a scaffold with a tubular film over a scaffold.
FIG. 19A depicts an axial projection of a tubular polymeric structure positioned over a tubular mandrel.
FIG. 19B shows struts of the scaffold pressed against the outer surface of a polymeric structure.
FIG. 20A depicts a section of a scaffold with a polymeric layer disposed within a gap between struts of the scaffold.
FIG. 20B depicts a cross section from FIG. 20A.
FIG. 21 depicts two adjacent axial segments of a scaffold in which a "Z" shaped flexible link connects adjacent rings.
FIG. 22A depicts an "S" shaped flexible link.
FIG. 22B depicts a single loop shaped flexible link.
FIG. 23 depicts two adjacent axial segments of a scaffold connected by "Z" shaped flexible links in which each peak and valley of adjacent rings of each axial segment is connected by link struts.
FIG. 24A depicts an axial scaffold segment with a pattern similar to that of FIG. 6A in an as-cut state.
FIG. 24B depicts the axial scaffold segment of FIG. 24B in a crimped stated.
FIG. 25A depicts an axial scaffold segment in an as cut state with a pattern similar to that of FIG. 6A which additionally includes keyhole features as depicted in FIG. 6D.
FIG. 25B depicts the scaffold segment of FIG. 25A in a crimped state.
FIG. 26A depicts six scaffold segments disposed over a balloon prior to crimping.
FIG. 26B depicts a close-up of one segment from FIG. 26A after crimping illustrating the balloon pillowing between scaffold segments.
FIG. 26C depicts five scaffold segments of the segment of FIG. 26A after crimping.
FIG. 27 depicts an exemplary axial scaffold segment with a pattern as shown in FIG. 6C.
FIG. 28A depicts the radial strength of a PLLA segmented scaffold and the radial strength of a PLLA non-segmented scaffold.

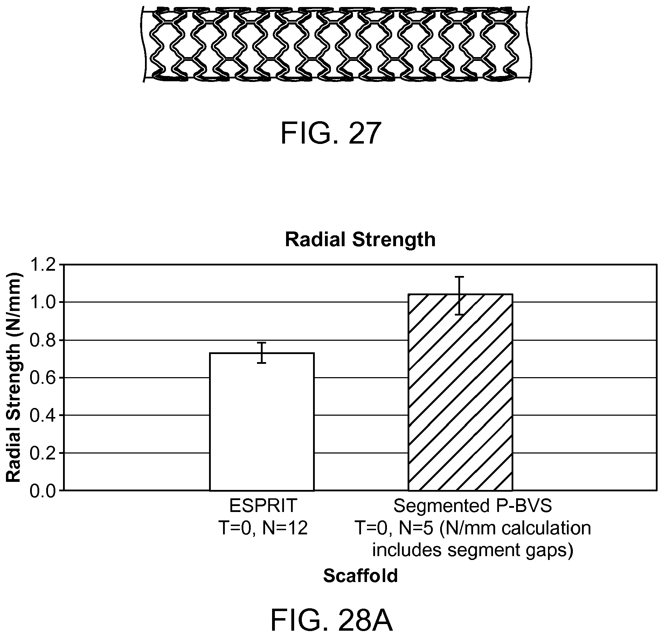

FIG. 28A depicts the radial stiffness of a PLLA segmented scaffold and the radial strength of a non-segmented scaffold.

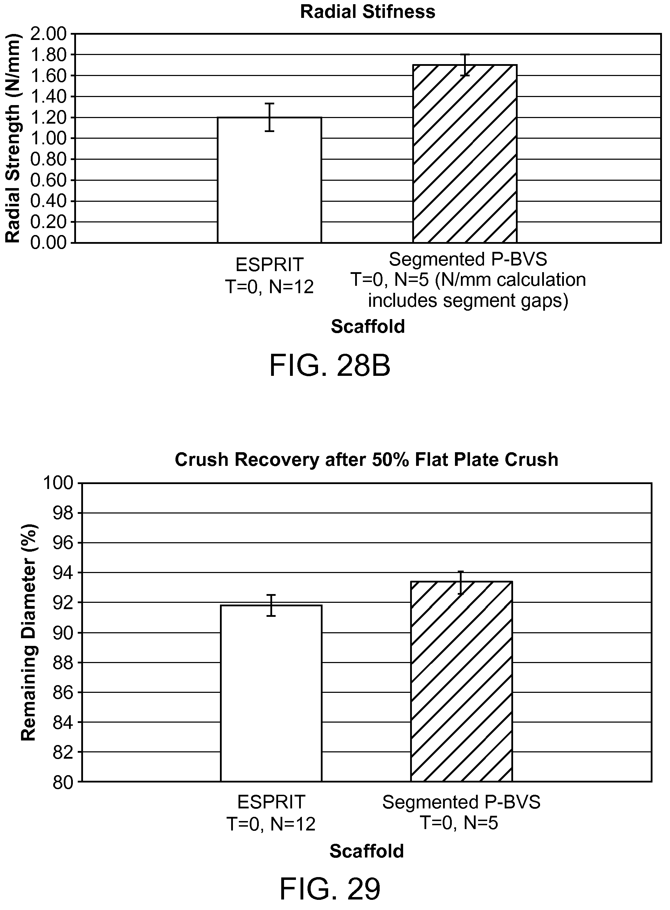

FIG. 29 depicts the crush recovery of the segmented scaffold and segmented scaffold after 50% crush.

### DETAILED DESCRIPTION OF THE INVENTION

Coronary arteries refer generally to arteries that branch off the aorta to supply the heart muscle with oxygenated blood. Peripheral arteries refer generally to blood vessels outside the heart. In both coronary artery disease and peripheral artery disease, the arteries become hardened and narrowed or stenotic and restrict blood flow. In the case of the coronary arteries, blood flow is restricted to the heart, while in the peripheral arteries blood flow is restricted leading to the kidneys, stomach, arms, legs, feet, and brain. The narrowing is caused by the buildup of cholesterol and other material, called plaque, on their inner walls of the vessel. Such narrowed or stenotic portions are often referred to as lesions. Arterial disease also includes the reoccurrence of stenosis or restenosis that occurs after an angioplasty treatment. Although there are probably several mechanisms that lead to restenosis of arteries, an important one is the inflammatory response, which induces tissue proliferation around an angioplasty site. The inflammatory response can be caused by the balloon expansion used to open the vessel, or if a stent is placed, by the foreign material of the stent itself.

In embodiments of the present invention, a stent, a stent scaffold, or scaffold includes a plurality of cylindrical rings connected or coupled with linking elements. When deployed in a section of a vessel, the cylindrical rings are load bearing and support the vessel wall at an expanded diameter or a diameter range due to cyclical forces in the vessel. Load bearing refers to the supporting of the load imposed by radial inwardly directed forces. Structural elements, such as the linking elements or struts, are non-load bearing, serving to maintain connectivity between the rings. For example, a stent may include a scaffold composed of a pattern or network of interconnecting structural elements or struts.

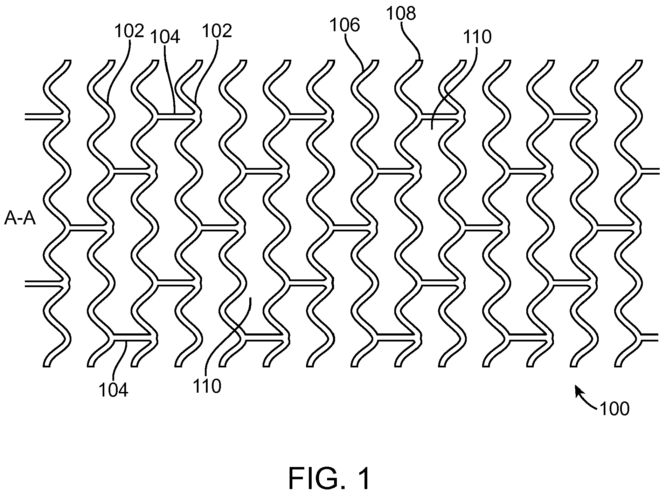

FIG. 1 illustrates a portion of an exemplary stent or scaffold pattern 100. The pattern 100 of FIG. 1 represents a tubular scaffold structure so that an axis A-A is parallel to the central or longitudinal axis of the scaffold. FIG. 1 shows the scaffold in a state prior to crimping or after deployment. Pattern 100 is composed of a plurality of ring struts 102 and link struts 104. The ring struts 102 forms a plurality of cylindrical rings, for example, rings 106 and 108, arranged about the cylindrical axis A-A. The rings are connected by the link struts 104. The scaffold comprises an open framework of struts and links that define a generally tubular body with gaps 110 in the body defined by rings and struts. The cylindrical tube of FIG. 1 may be formed into this open framework of struts and links described by a laser cutting device that cuts such a pattern into a thin-walled tube that may initially have no gaps in the tube wall.

The structural pattern in FIG. 1 is merely exemplary and serves to illustrate the basic structure and features of a stent or scaffold pattern. A stent such as stent 100 may be fabricated from a polymeric tube or a sheet by rolling and bonding the sheet to form the tube. A tube or sheet can be formed by extrusion or injection molding. A stent pattern, such as the one pictured in FIG. 1, can be formed on a tube or sheet with a technique such as laser cutting or chemical etching. The stent can then be crimped on to a balloon or catheter for delivery into a bodily lumen.

The width and or thickness of the struts in a scaffold may be 100 to 200 microns, or more narrowly, 130 to 180 microns, 140 to 180 microns, or 140 to 160 microns.

Semicrystalline polymers such as poly(L-lactide) (PLLA) with glass transition temperature (Tg) above human body temperature are suitable as materials for a totally bioabsorbable scaffold since they are relatively stiff strong at the conditions of the human body. However, they tend to be brittle at these conditions. These polymer systems exhibit a brittle fracture mechanism in which there is little or no plastic deformation prior to failure. As a result, a stent fabricated from such polymers can be vulnerable to fracture during of use of a scaffold, i.e., crimping, delivery, deployment, and during a desired treatment period post-implantation.

Embodiments of the present invention are applicable to endovascular treatment of coronary and peripheral disease in coronary arteries and various peripheral vessels including the superficial femoral artery, the iliac artery, and carotid artery. The embodiments are further applicable to various stent types, such as self-expandable and balloon expandable stents. The embodiments are further applicable to various stent designs including scaffolding structures formed from tubes, wire structures, and woven mesh structures.

In general, the initial clinical need for a bioabsorbable scaffold is to provide mechanical support to maintain patency or keep a vessel open at or near the deployment diameter. The scaffold is designed to have sufficient radial strength to maintain such patency for a period of time. The patency provided by the stent allows the stented segment of the vessel to undergo healing and remodeling at the increased diameter. Remodeling refers generally to structural changes in the vessel wall that enhance its load-bearing ability.

A period of patency is required in order to obtain permanent positive remodeling and vessel healing. However, the vessel requires the patency for only a finite time to obtain such positive remodeling. As the polymer of the stent degrades, the radial strength of the scaffold decreases and the load of the vessel is gradually transferred from the scaffold to the remodeled vessel wall. In addition to the decline in radial strength, the degradation of the scaffold also causes a gradual decline in the mechanical integrity. Mechanical integrity refers to the connectivity of struts and the size and shape of the overall scaffold structure. The struts gradually resorb and disappear from the vessel.

The amount of movement experience a peripheral scaffold is greater than what a coronary scaffold experiences in the coronary artery. A peripheral scaffold can be subjected to a high degree of flexing, axial elongation/compression, pinching, bending, and torsion after implantation. Axial stresses on the scaffold can arise from the axial compression and extension, flexural stresses are imposed by lateral flexing, crushing forces are imparted by pinching, while helical stress can arise from torsional forces.

Such stresses are propagated along the length of the scaffold and can impart significant stress and strain throughout the scaffold structure. The stresses can results in failure of link struts which can cause instability in the rings if the rings are not sufficiently endothelialized in the vessel wall. The stability refers to the ability of the ring to resist tipping or rotating within the vessel. In addition, these forces can cause failure in ring struts as well. Such forces can be transmitted along the length of the scaffold by link struts that connect rings.

Strut breakage is not inherently deleterious to either performance or safety. Bench testing and animal study results suggest that scaffold properties of radial strength, crush recovery, and crush resistance are primarily attributable to the integrity of the rings in the scaffold and not the links.

Strut breakage can also lead to release of fragments in the blood and tissue irritation from broken strut fragments. Fragment release could result in thrombosis. Broken fragments can be mechanically injurious to the vessel leading to tissue irritation or even vessel dissection and perforation.

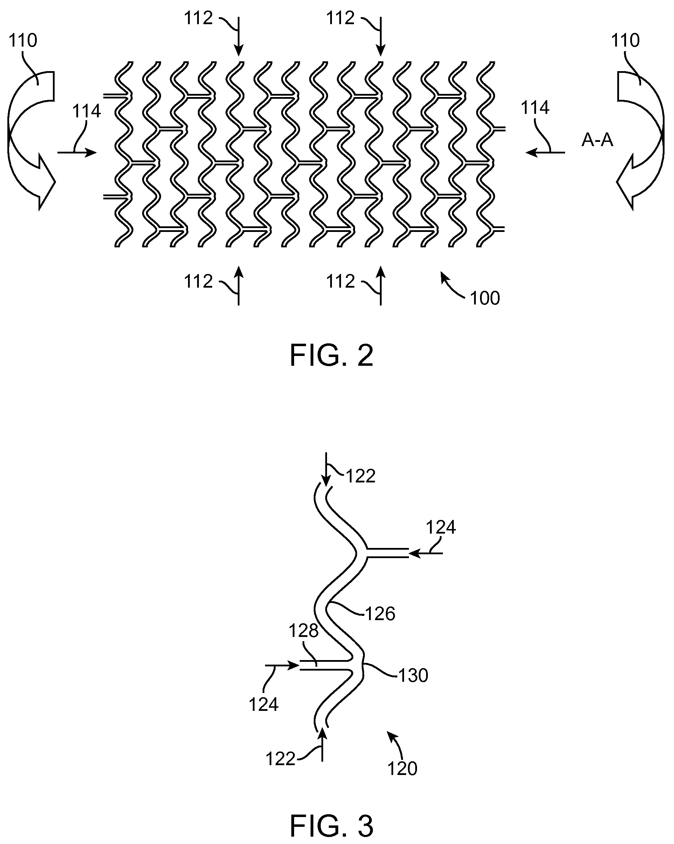

FIG. 2 depicts the exemplary scaffold pattern 100 which shows schematically the forces acting on the scaffold. Line A-A represents the cylindrical axis of the stent. The arrows around the edges represent the forces acting on the scaffold during delivery and after deployed. Arrows 110 represent bending, arrows 112 represent radial compression, and arrows 114 represent axial compression. Bending occurs during delivery through torturous anatomy and to a lesser extent after deployment. Radial and axial compression occurs after deployment.

Cracks in the scaffold occur when it is subjected to a sufficiently high force such as resulting from bending during delivery or repetitive forces after deployment that cause fatigue. These cracks can cause a loss of radial strength or separation of parts of the scaffold that drift downstream of the scaffold.

FIG. 3 depicts a strut section 120 of pattern 100 of FIGs. 1 and 2. The arrows in FIG. 3 represent the forces acting on this section of the scaffold pattern. The strut section is shown in a deployed configuration, but the same stent when collapsed under bending can be envisaged. Radially compressive forces on the scaffold caused by the push back of the vessel walls on the scaffold are represented as arrows 122. Arrows 124 are from axial compressive forces which, in the SFA, arise due to movement of a leg such as during walking or bending of the leg. In the SFA, the axial compressive forces can be considerable as the vessel is compressed up to 7% or more and relaxed repeatedly up to one million cycles/year.

Referring again to FIG. 3, locations 126, 128, and 130 represent areas where cracks are observed to occur in a scaffold from use. A crack in the ring, i.e., at 126 or 130 will cause a loss of radial strength, while a crack in the link at 128 is less damaging to the scaffold in terms of radial strength, crush resistance, and crush recovery. It is believed that if the axial forces on the scaffold were reduced, the occurrence of ring cracks would be significantly reduced. It follows that the negative impact of vessel forces on the radial strength, crush recovery, and crush resistance of the scaffold would be significantly reduced.

The various embodiments of the present invention are directed to improving the performance of peripheral scaffolds subject to significant nonpulsatile forces upon implantation. Some embodiments are directed to reducing or eliminated the negative effects of scaffold properties from strut fracture and breakage. Other embodiments additionally reduce the degree of strut fracture and breakage.

Some examples are particularly applicable to a scaffold with cylindrical rings of struts connected by link struts, such as the exemplary scaffold described in FIGs. 1-3. The examples include modifications that improve the performance of three general classes of scaffolds. The present invention further includes the third class of scaffolds, whose structural features reduce fractures, breakage, and failure of struts, particularly ring struts.

The first class includes scaffolds composed of cylindrical rings connected by link struts that are not designed to selectively fracture or break. Scaffold pattern 100 is an example of such a pattern. Although link struts may be fracture, break, or fail due to the forces described above, specific link struts or sets of link struts are not designed to preferentially fracture, break, or fail over other link struts. Examples of such patterns are disclosed in US Patent Publication US20110190872.

The second class of scaffold includes link struts or specific sets of link struts that are preferentially designed to fail over other linking struts. For example, all the linking struts between selected pairs of rings may be designed to preferentially fail at some time after implantation. The selected pairs of rings can be selected so that after failure the scaffold includes disconnected sets of rings between the sets of link struts that are designed to fail. When the links fail after implantation, the scaffold includes decoupled axial segments that are no longer connected. Since the axial segments are no longer connected, axial compression on the segments are not transmitted to the other segments, which reduces fracture and failure of ring struts.

Link struts can be preferentially designed to fail, for example, by a structural feature that weakens the strut at a location or region on the strut that makes it more susceptible to fracture and failure. For example, the strut can have a notch at a location which weakens the strut. Examples of scaffolds that have struts that are preferentially designed to fail are disclosed in US20110066225, U.S. Patent Application No. 12/882,978, US20110190872, and US20110190872.

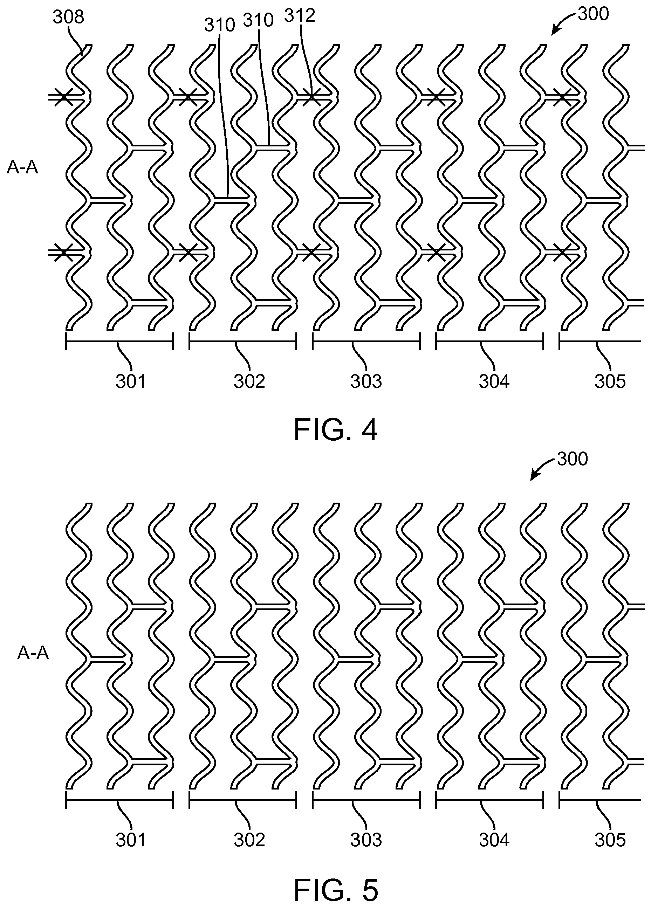

FIG. 4 depicts a scaffold 300 composed of rings 308 of struts connected by link struts 310. Selected linking struts between every third or fourth ring have weakened portions 312 represented by an "X" between every third. Axial scaffold segments 301 to 305 decouple or are separated when the link struts selectively fail upon implantation.

The third class of scaffolds is composed of axial scaffold segments that are not connected by link struts. Before discussing modifications applicable to the three classes of scaffolds that improve their performance, the examples of the third class of scaffolds will be described in detail. Examples of such a scaffold include two or more radially expandable axial scaffold segments arranged axially end to end. The axial segments, in particular, axially adjacent segments are not connected by any physical structure or material of the scaffold. The axial segments, however, may be indirectly in contact through another structure such as a support member or a sheath.

In general, upon deployment of the scaffold segments, forces subjected on one axial segment cannot be transmitted to other axial segments. The axial segments may be composed of a plurality of interconnected struts. Forces subjected to a segment can be transmitted between struts within the segment, but not between segments.

In some embodiments, the axial segments are composed of one or more cylindrical rings of struts. A cylindrical ring may be composed of undulating struts having crests and troughs. The cylindrical rings of struts that are adjacent in a segment are connected. The rings may be connected by link struts. Alternatively, the rings may be directly connected to one another without link struts. The number of rings in a segment may be one or any number greater than one. In some embodiments, a segment can have 1 or more, 2 or more, 1 to 6 rings, 1 to 3 rings, 2 to 6 rings, or 2 or 3 rings.

Upon deployment, the axial segments remain intact for a period of time and maintain a ring shape at or near the deployed diameter. Since the axial segments are not connected, they are decoupled which prevents transmission of axial compression between segments. The decoupled axial segments retain sufficient radial strength to support the vessel at or near the deployed diameter. The decoupling of the axial segments reduces stress, for example, from axial compression that causes failure of ring struts. The reduced ring strut fracture helps maintain the radial strength and the crush recovery and resistance of the scaffold. The decoupling of rings reduces or prevents propagation of failure to rings due to bending of the stent structure along its axis.

In some examples a scaffold with decoupled axial segments can be fabricated by forming the axial segments separately. For example, a scaffold pattern can be cut into a thin-walled tube having an axial length the same as the desired axial segment. Alternatively, a scaffold can be fabricated from by laser cutting a tube and then axial segments can be formed cutting the scaffold into disconnected axial segments by cutting the link struts or cutting the link struts off entirely.

Referring again to scaffold 300 in FIG. 4, disconnected or decoupled axial scaffold segments 301 to 305 can be formed by cutting or removing link struts 312 identified by the "X." FIG. 5 depicts scaffold 300 after removal of link struts 312 showing disconnected axial segments 301 to 305. Alternatively, axial segments 301 to 305 can be formed separately by cutting up a larger scaffold into several axial segments. The separation of scaffold 300 into several axial segments interrupts the compressive forces on the scaffold which greatly reduces their contribution to scaffold cracking.

The stability of an axial segment depends on the width of the axial segment. The stability is inversely related to the width of the axial section. The susceptibility to fracture, however, is directly related to the width of the axial section. The width of the axial segments should be large enough so that it has a desired stability.

The radial strength and radial stiffness of a scaffold or scaffold segment increases with the degree of connectivity of a scaffold. The degree of connectivity refers in part to the number of link struts between rings and the length of the link struts: more link struts and shorter link struts tend to increase strength and stiffness. The stiffer the scaffold, the more susceptible the scaffold is to fracture. In the present examples since compressive forces are not transmitted to along an entire scaffold length, the scaffold segments can be made with a higher connectivity than a scaffold that does not have disconnected axial segments.

In the scaffold axial segments such as those depicted in FIG. 5 the crests of the axial rings are axially aligned or approximately axially aligned. The stiffness of the axial segments of such a scaffold can be increased by increasing the number of link struts between axially adjacent peaks of adjacent rings. Every pair of aligned peaks between adjacent rings can be connected, every other pair of aligned peaks can be connected, or every third pair of aligned peaks can be connected by a link strut.

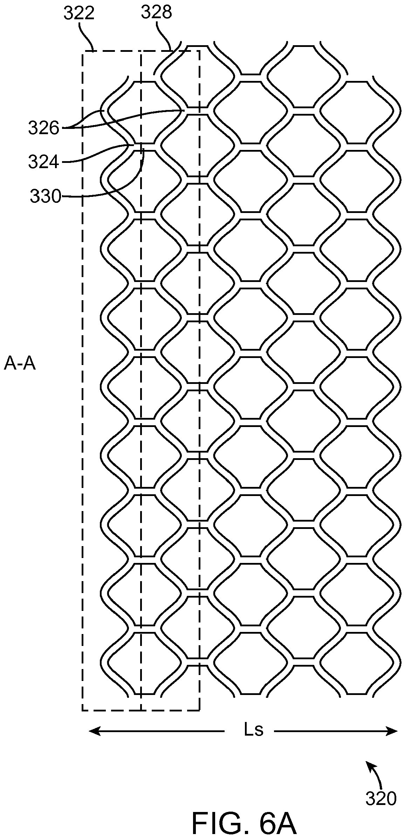

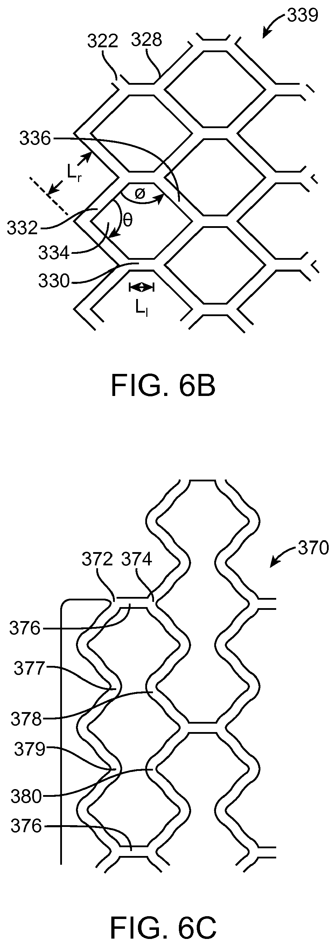

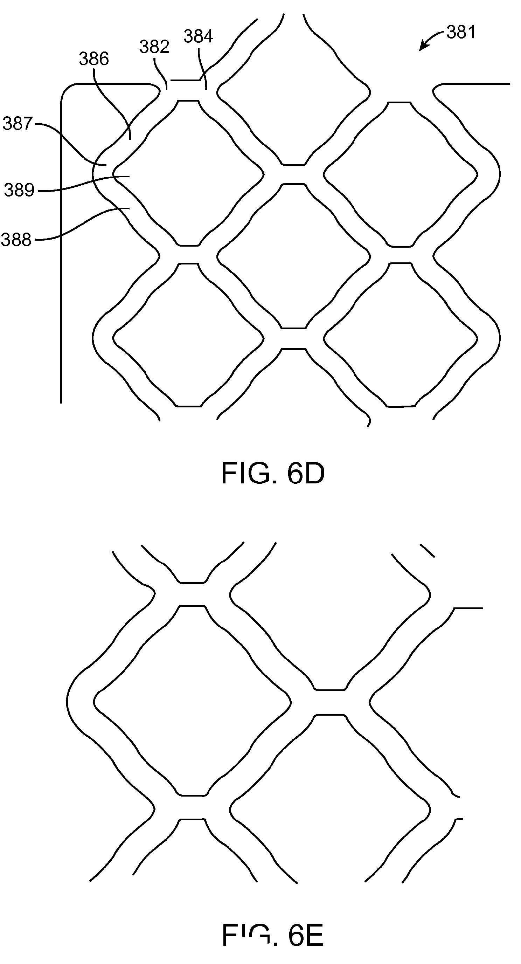

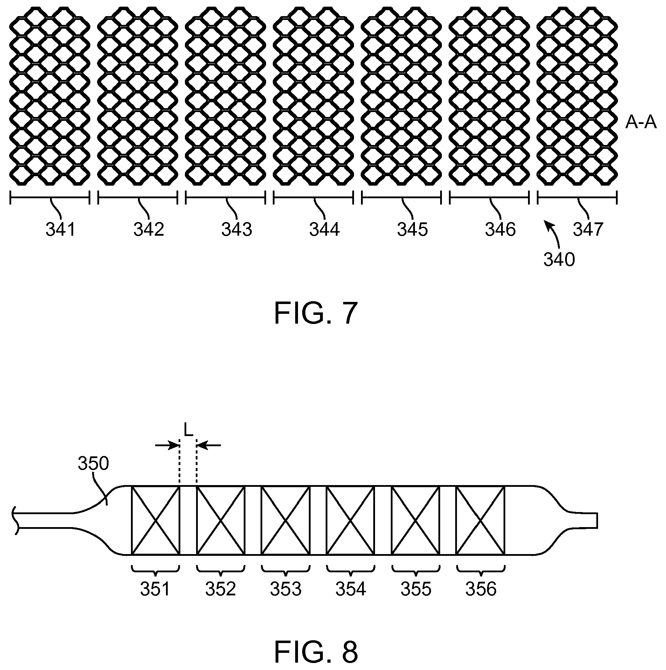

The axial segments are composed of rings arranged such that the crests in one ring are axially aligned or almost axially aligned with the troughs in an adjacent ring. The rings are connected by at least one link strut between an aligned crest and trough. Stiffness is greatest with a link strut between each aligned crest and trough. Greater flexibility is introduced by having fewer than every aligned crest and trough connected by a link strut. For example, only every other aligned crest and trough can be connected, or only every third aligned crest and trough can be connected by a link strut. Additionally, the length of the link struts in the axial segments can be adjusted to modify the stiffness of the axial segment. Decreasing the length of the links increases both the radial strength and radial stiffness of the axial segment since the number of rings per segment length is maximized. Such a pattern may also be described as a plurality of rings composed of diamond-shaped elements formed of struts. The elements of the rings are connected at circumferentially aligned vertices of the diamond-shaped elements. Axially adjacent rings are connected at axially aligned vertices either by a short link strut or at the intersection of vertices of elements of adjacent rings.FIG. 6A depicts an exemplary axial segment 320 viewed in a flattened configuration composed of a plurality of rings of undulating struts with crests and troughs. Line A-A is the longitudinal axis of the axial segment. An exemplary ring 322 has crests 324 and troughs 326. As shown in FIG. 6, every crest in ring 322 is connected to every trough in adjacent ring 328 by a short link strut 330. Ls is the length of the axial segment. Ls may be 3 to 6 mm, 6 and 8 mm, 8- 10 mm, 10 to 12 mm, or greater than 12 mm. FIG. 7 depicts a scaffold 340 composed of a plurality of axial segments 341 to 347, from FIG. 6A.

FIG. 6B depicts a close-up view of a portion 339 of axial segment 320 illustrating various features. As shown in FIG. 6B, Lr is the length of a ring strut, for example, strut 332 between a crest and trough in a ring and Wᵣ is the width of the ring strut. Lₗ is the length of short link strut 330 that connects a crest and trough of adjacent rings and Wₗ is the width of the link strut. θ is the angle between struts 332 and 334 in a ring that intersects at a crest or trough. ϕ is the angle between struts 332 and 336 which are joined by short link strut 330 and which form an opposing portion of a diamond-shaped cell. Hc is the height of the diamond-shaped cell and Wc is the width of the diamond-shaped cell.

θ may be 90 degrees, 90 to 95 degrees, 95 to 100 degrees., 100 to 110 degrees, or greater than 110 degrees. θ may be 90 degrees, 85 to 90 degrees, 80 to 85 degrees., 70 to 80 degrees, or less than 70 degrees. ϕ may be 90 degrees, 85 to 90 degrees, 80 to 85 degrees., 70 to 80 degrees, or less than 70 degrees. ϕ may be 90 degrees, 90 to 95 degrees, 95 to 100 degrees., 100 to 110 degrees, or greater than 110 degrees.

Lₗ may be less than 10% or 10% to 20%, 20% to 30%, 30 to 40%, or greater than 40% of a ring strut length between a crest and a trough. Exemplary link struts may have a length of less than 0.01 in, 0.01 to 0.02 in, 0.02 to 0.04 in, or 0.04 to 0.06 in, or greater than 0.06 in. In some embodiments, adjacent rings are connected at an intersection of the opposing crests and troughs such that a length of the link strut is effectively the width of the intersection.

FIG. 6C depicts a portion 370 of another exemplary pattern of an axial scaffold segment having fewer than every aligned crest and trough of adjacent rings 372 and 374 connected by a short link strut. Specifically, only every third aligned crest and trough is connected by short link struts 376. Crest and trough 377, 378 and 379, 380 are not connected by a short link strut. Lₗ for portion 370 is greater than Lₗ for portion 339 of 6B to avoid ring to ring interference. Lₗ for portion 370 is greater than 20 or 30% of Lr.

FIG. 6D depicts a portion 381 of another exemplary pattern of an axial scaffold segment with keyhole features at the inner surface of the crests and troughs. Portion 381 includes rings 382 and 384 with a trough 387 formed by links 386 and 388. Trough 387 has a keyhole feature 389 which is an indentation at its inner surface.

FIG. 6E depicts a close-up portion of the exemplary pattern of FIG. 6D with exemplary dimensions for θ, ϕ, H_{c}, W_{c}, Wᵣ, Wₗ, and Lₗ. The lengths are in inches. Additional variations can be ±20° for the angles, ±.040 in on the strut lengths, ±.005 in on the strut thickness. Links may vary from 0 to .050 in. These dimensions can apply to any of the diamond shaped axial segment patterns.

The axial segments may further include radiopaque which may be gold or Platinum foil wrapped around the end strut or link

The delivery of a scaffold composed of decoupled axial segments, such as that shown in FIG. 5 and 7 can be achieved by disposing the axial segments on a delivery device. The axial segments can be arranged end to end and spaced apart on a single balloon. The axial segments may be crimped over the balloon to a reduced diameter configuration to allow for delivery to a vascular system to a treatment site. FIG. 8 depicts a cross section of axial segments 351 to 356 disposed over a balloon 350 in a deflated configuration. Axial segments are crimped tightly over the balloon in a reduced diameter configuration. The axial segments are spaced apart by a distance L. The distance between segments preferably should be the at least, the same as, or close to (e.g., within 5 or 10% of) the spacing between rings within a segment. In this way the scaffold pattern approximately continues between segments. As a result, the degree of vessel wall support is approximately continuous. However when compressive loads are placed on the scaffold the compression may occur predominantly between segments. From bench testing it has been shown that a distance of 1mm or more is preferred to allow for the decrease in the spacing of the segments during compression and loading, in general. Preferably, the segment ends should not collide during bodily movements. In exemplary embodiments, the segments are spaced apart 0.5 to 2mm, or more narrowly, 0.5 to 1 mm, or 1 to 2 mm.

In some embodiments, the segmented scaffold is designed such that the segments individually have maximum radial strength and crush resistance. Current bench testing shows that the radial strength has been increased by more than 40% over non-segmented designs. With this improvement the potential for crush is greatly reduced.

The diamond pattern disclosed herein tends to maximize the relative friction between the vessel wall and the segments. With this and the high radial and axial rigidity of the diamond pattern, endothelization of the segments may be sped up and vessel irritation may be reduced. With quick endotheliization, the scaffold/vessel wall becomes a composite structure which in itself enhances the radial strength and hence crush resistance. With most, if not all of the movement transferred to the gaps between the segments, the design utilizes the natural flexibility of the vessel walls to handle any compression, bending and torsional movements. This has been confirmed with preliminary bench testing.

Various modifications may be made to the classes of scaffolds described herein to improve performance upon implantation. Embodiments of these modifications reduce the negative effects of fracture, breakage, or failure of struts for all three classes of scaffolds discussed herein. The modifications improve the stability of the scaffolds. With respect to the first and second class of scaffolds, segments separated by fractures and breakage can result in movement of separated segments with respect to one another. Instability of separated axial sections can also result. With respect to the third class of scaffolds, the embodiments stabilize the axial sections which are separated at the outset. Additionally, for all three classes of scaffolds, the modifications prevent release of fragments generated by fracture and breakage and also shield the vessel from broken struts, thereby preventing tissue irritation and damage.

The modifications may include a polymeric structure or structures bonded to the scaffold and extending along its length. The polymeric structure can be bonded to an outer surface (tissue contacting surface or abluminal surface) or the inner surface (luminal surface), or both. The polymeric structure can also be bonded to the side walls of the struts of the scaffold. The scaffold can also be partially or completely embedded in the polymeric structure. The polymeric structure is not part of the scaffold since it is not formed from laser cutting a pattern into a tube. As described in more detail below, the polymeric structure can include polymeric elongate elements, a fiber mesh tube, or a polymeric tubular film. In some embodiments, the polymeric structure is free of a drug or therapeutic agents, except for incidental diffusion from an adjacent drug-containing layer.

The polymeric structure extends across some or all of the gaps along the length of the stent. For example, the polymeric structure may extend across the gap between ring struts, ring struts and link struts, or two link struts. The polymeric structure coverage of the surface area of the gaps may be less than 10%, greater than 50%, 10 to 20 %, 20 to 50%, 50 to 70%, 70 to 90 % or greater than 90%. It is important for the gap surface area to be sufficiently porous to allow endothelial growth to occur that will cover the struts. The porosity can refer to the size or average size of the pores of the coverage. The porosity can also refer to the maximum size of particles or blood components that can pass through the polymeric structure covering the gaps.

The porosity of the polymeric structure coverage of the gaps can be adjusted to allow permeation or prevent (or limit) permeation of any blood components through the gap or cellular material from the vessel wall. For example, monocytes may be allowed to permeate through the gaps. The porosity of the polymeric structure coverage of the gaps can also be adjusted to allow or prevent permeation of scaffold fragments into the lumen. The blood component or fragment size that can permeate through the gaps can be limited to less 30 microns, 50 microns, 100 microns, 200 microns, 300 microns, or less than 500 microns.

The polymeric structure can be applied over a scaffolding that includes a medicated coating. Alternatively, the polymeric structure can be applied to a scaffolding with or without a medicated coating. After applying the polymer structure, a medicated coating can be formed over the scaffolding with the polymeric structure.

The polymeric structure may be made of polymer that is relatively flexible at human body conditions, such as those with a Tg below body temperature (about 37 degrees C) or below room temperature (e.g., between 20 to 30 degrees C). For example, the polymer may have an elongation at break greater than 10%, 20 %, 50%, or greater than 100%. The polymer may be characterized as an elastomer.

The flexibility of the polymeric structure will allow movement of decoupled segments or sections of a scaffold, which reduces or dampens the transmission of compressive forces between these sections. Thus, the polymeric structure can provide stability without or without significantly transmitting axial compressive forces along the scaffold length. Exemplary flexible polymers include polycaprolactone (PCL) and poly(trimethylene carbonate) (PTMC), polydioxanone (PDO), poly(4-hydroxy butyrate) (PHB), and poly(butylene succinate) (PBS). Additional flexible polymers further include random, alternating, or block copolymers including the above polymers. For example random or block copolymers with PLLA and PGA, for example, PLLA-b-PCL, PLLA-b-(PGA-co-CL), or PLLA-co-PCL. Additional flexible polymers further could be a physical mixing of the above polymers or mixing with additives known in the art to achieve desired properties.

Upon implantation, the structure stabilizes the scaffold since it maintains a connection between axial segments of the scaffold that separate due to fracture of linking struts. The structure also prevents release of broken strut fragments from the scaffold and shields the tissue from irritation and injury from these broken struts.

In some examples the polymeric structure includes a plurality of polymeric elongate elements bonded to the surface of the struts of the scaffold. An elongate element is a structure with a length much longer its width (e.g., length is more than 5, 10, 20, or more than its width or diameter). The polymeric elongate elements may be cords, ribbons, or fibers. A fiber may have diameter or a ribbon may have a width of less than 30 microns, 30 to 50 microns, 50 to 80 microns, 80 to 100 microns, 100 to 150 microns, or greater than 150 microns. A ribbon may have a thickness of less than 20 microns, 20 to 50 microns, or 50 to 100 microns. A ribbon may be disposed on the scaffold with its wider side in contact with the scaffold.

The elongate elements extend along the length of the scaffold and across gaps between struts of the scaffolding. The axis of the elongate elements refers to the orientation of the element along its length. The elongate elements may be bonded to the surface of the adjacent axial segments. In some examples, the elongate elements are bonded to an outside (abluminal) surface of the scaffold, inside (luminal) surface of the scaffold, or both.

The polymeric elongate elements can be arranged on the axial segments in a variety of ways. The elongate elements can be arranged parallel to the cylindrical axis of the axial segments, i.e., ranged axially or longitudinally. Alternatively, the elongate elements can be arranged at an angle to the cylindrical axis. For example, the elongate element axis may be at an angle with an absolute value less than 90, 80, 70, 60, 30, 20, or 10 degrees with respect to the axis of the scaffold. It is preferable to have elongate elements with an axial component (less than 90 degrees relative to the scaffold axis) in order to provide stability to decoupled axial segments or strut fragments that are axially distal to one another.

The elongate elements can extend across gaps between struts in the scaffold. The elongate elements can have flex or slack in the portion of the elements that extend across the gaps. The surface area of the gaps may be only partially covered by elongate elements extending across the gaps. The surface area of the gaps that is covered by the elongate elements over the gaps can be adjusted by the width or diameter of the elongate elements and/or the number of elongate elements across the gaps. The porosity of the gaps can also be adjusted by the same variables. The number of elongation elements and/or their thickness across gaps can be adjusted to allow or limit size of cells or particles that can permeate through the gaps in the ranges discussed above.

The flexibility of the elongate segments reduces or dampens the transmission of axial forces between adjacent axial segments that have separated. The elongate segments also assist in the stabilization of the scaffold segments. Since shorter axial sections are less stable, the elongate elements allow use of shorter axial segments in the second and third classes of scaffolds. This is an advantage since shorter segments are less susceptible to fracture. The resulting scaffold with the elongate elements may be highly radially rigid, yet flexible in the axial and longitudinal bending directions.

The degree of stabilization provided by the elongate elements can be adjusted in a number of ways. The stabilization can be increased by using a polymer with a higher modulus. Also, as the number or density of the elongate elements on the scaffold increases, the stabilization increases. The thickness of the elongate elements can be adjusted to control the stabilization; the thicker the elements, the greater the stabilization.

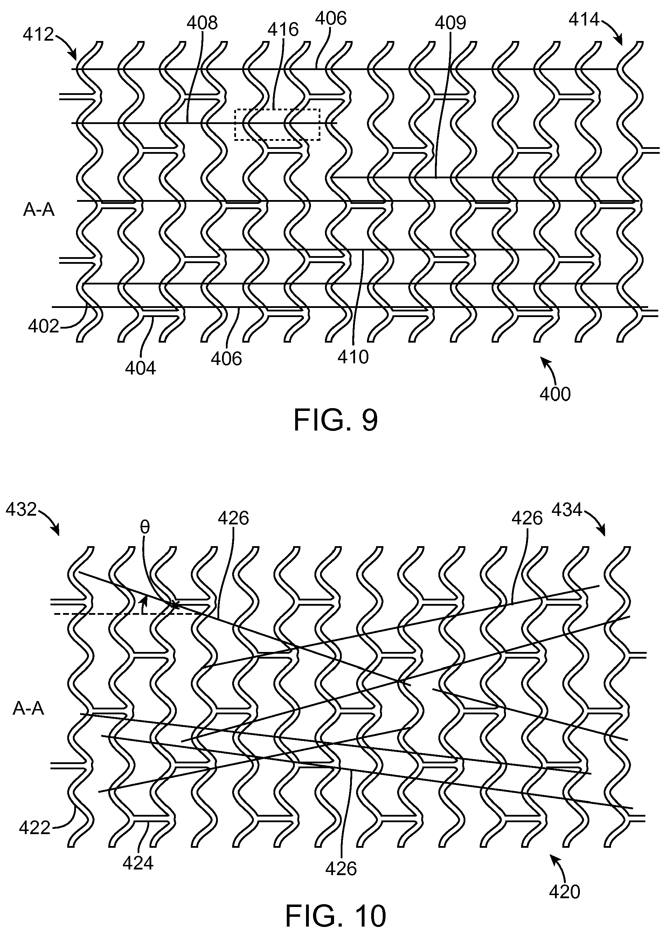

FIG. 9 depicts a scaffold pattern 400 that is composed of rings 402 connected by link struts 404. Scaffold pattern 400 has a proximal end 412 and a distal end 414. Line A-A corresponds the axis of pattern 400. The pattern corresponds to the first class of scaffolds discussed above without weakened links. However, the example shown in FIG. 9 is equally applicable to the second class of patterns with weakened links. FIG. 9 shows elongate elements or fibers, for example, fibers 406, 408, 409, and 410 bonded to the outer surface of the struts of pattern 400. The axis of the fibers is parallel with the axis of the scaffold. Fibers such as fiber 406, extend between the proximal end to the distal end of pattern 400. Other fibers such as fibers 408 and 409 extend from proximal end 412 to an intermediate location along the pattern or extend from distal end 414 to an intermediate location along the pattern, respectively. Additionally, fibers such as fiber 410 extend along an intermediate section without extending to either the proximal or distal end of the pattern.

FIG. 10 depicts a scaffold pattern 420 that is composed of rings 422 connected by link struts 424. Scaffold pattern 400 has a proximal end 432 and distal end 434. Line A-A corresponds to the axis of pattern 420. FIG. 10 shows elongate elements or fibers 426 bonded to the outer surface of the struts of pattern 420. The axis of the fibers is not parallel with the axis of the scaffold and is at an angle θ greater than 0 with respect o the axis of the scaffold.

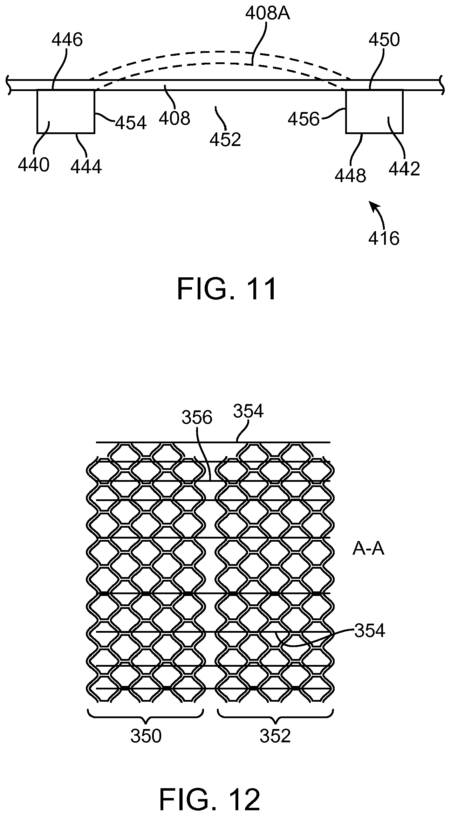

FIG. 11 depicts a cross section of a section 416 of pattern 400 in FIG. 9. FIG. 11 depicts strut 440 and strut 442. Strut 440 has an inner surface 444 and an outer surface 446. Strut 442 has inner surface 448 and an outer surface 450. Fiber 408 is bonded to outer surface 446 of strut 440 and outer surface 450 of strut 442. Fiber 408 extends across gap 452 between the struts. Fiber 408A shown in dashed lines is an alternative depiction of a fiber that is flexed outward across gap 452 between struts 440 and 442. In another example (not shown) the fiber can be bonded to at least part of side wall 454 of strut 440 and side wall 456 of strut 442.

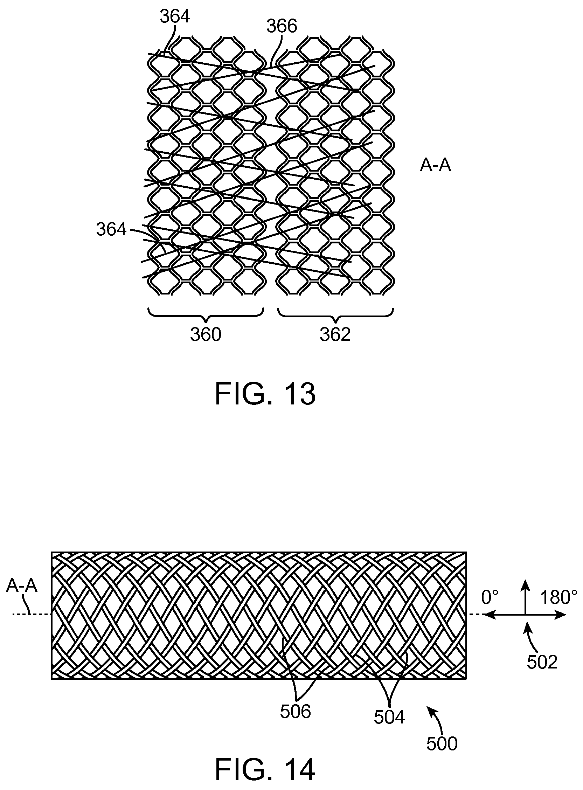

FIGs. 12 and 13 depict polymeric elongate elements bonded to a surface of decoupled axial segments. FIG. 12 depicts axial segments 350 and 352 that include polymeric elongate elements 354 bonded to a surface of axial segments 350 and 352. Elongate elements 354 are arranged parallel to the axis (A-A) of the axial segments 350 and 352. Elongate elements extend across the surface of axial segments 350 and 352 across gap 356 between the axial segments.

FIG. 13 depicts axial segments 360 and 362 that include polymeric elongate elements 364 bonded to a surface of the axial segments. Elongate elements 364 are arranged at various angles to the axis (A-A) of the axial segments. Elongate elements 364 extend across the surface of axial segments 360 and 362 across gap 366 between the axial segments.

In some examples the elongate elements extend only between adjacent axial segments. In order to increase the stabilization provided by the elongate elements, in other examples the elongate elements can extend between more than two axial segments, such as any number of axial segments. In some examples, elongate elements extend between a proximal to a distal axial segment of a scaffold that is to be delivered in a vessel.

The elongate elements can be bonded to the surface of a scaffold in a variety of ways. In some examples the elongate elements can be deposited and bonded to a scaffold using electrospinning. Electrospinning refers to a process in which a high voltage is used to create an electrically charged jet of polymer fluid, such as a polymer solution or melt, which dries or solidifies to leave a polymer fiber. A system for electrospinning can include a syringe, a nozzle, a pump, a high-voltage power supply, and a grounded collector. An electrode is placed into the polymer fluid or attached to the nozzle and another electrode can be attached to a grounded collector.

The polymer fluid is loaded into the syringe and the liquid is driven to the catheter tube tip by the syringe pump, forming a droplet at the tip. An electric field is subjected to the end of the catheter tube that contains the polymer fluid, which is held by its surface tension. The field induces a charge on the surface of the liquid. Mutual charge repulsion causes a force directly opposite to the surface tension.

As the intensity of the electric field is increased, a charged jet of fluid is ejected from the tip of the catheter. The jet is then elongated and deposited on the grounded collector. The fiber tends to lay itself in an irregular or random fashion on the grounded collector.

In the present examples the scaffolds described above can be positioned over a tubular support. Fibers can be deposited on the scaffold using electronspinning. Fibers are deposited on the scaffold with a fiber axis with an axial component or that extend axially along the scaffold surface by translating the spinneret along the cylindrical axis of the scaffold. In the case of a decoupled scaffold, the fibers are deposited such that they extend between adjacent axial segments by translating the spinneret along the cylindrical axis of the axial segments. The support member can also be rotated to deposit such fibers around the circumference of the scaffold. Alternatively, the support member can be translated longitudinally with respect to the spinneret.

The deposited elongate segments may be bonded to the surface of the scaffold by solvent bonding or with an adhesive. The deposited fibers from electrospinning may have residual solvent that can partially dissolve or swell the polymer of the scaffold. Additionally, a solvent that is the same or different than is used for the electronspinning solution may be applied to partially dissolve or swell the polymer surface of the scaffold. The residual solvent is removed by evaporation or drying resulting in the fibers being bonded to the scaffold.

Exemplary solvents that can be used for electrospinning and solvent bonding in general include acetone, ethanol, ethanol / water mixtures, cyclohexanone, chloroform, hexafluoroisopropanol, 1,4-dioxane, tetrahydrofuran (THF), dichloromethane, acetonitrile, dimethyl sulfoxide (DMSO), N,N-dimethylformamide (DMF), N,N-dimethylacetamide (DMAC), cyclohexane, toluene, methyl ethyl ketone (MEK), xylene, ethyl acetate, and butyl acetate.

In other examples the fabrication of the elongate elements and application and bonding can be performed as separate steps. The elongate elements can be made by various methods known in the art such as by electrospinning or fiber spinning. The elongate elements can then be applied to the surface of the scaffold in a desired configuration along the scaffold.

In some examples the elongate elements are applied and bonded to the scaffold when the scaffold is in an as-cut or expanded configuration. For example, the as-cut diameter may be 3 to 4 mm for a coronary stent, 5 to 7 mm for an SFA stent, and 6 to 12 mm for an iliac stent. The scaffolds discussed herein increase in length as their diameter is reduced to a crimped configuration due to bending of the undulating rings. The crimped configuration may 2 to 5 mm. Therefore, as a scaffold is crimped, the elongate elements are placed into tension along the longitudinal axis. When the scaffold is deployed at delivery, the tension is relieved.

In other examples the elongate segments are applied and bonded to a scaffold when in a reduced diameter configuration. The scaffold decreases in length when the diameter is increased to a deployed configuration due to bending of the undulating rings. Therefore, as a scaffold with the elongate elements is deployed, the elongate elements will have flex or slack between gaps.

In further examples, the elongate elements are applied and bonded to the scaffold at a diameter between as-cut (De) or expanded configuration (Dc). For example, the elongate elements are applied at a D = Dc + X (De - Dc), where X can be 0 to 0.2, 0.2 to 0.4, 0.4 to 0.6, 0.6 to 0.8, 0.8 to 1. The tension in the elongate elements along the scaffold axis is reduced when the scaffold is crimped to the reduced delivery diameter as compared to applying the elongate elements in the fully expanded as-cut configuration. Additionally, when the scaffold is deployed, the degree of slack or looseness in the elongate elements is less than when the elongate elements are applied in the fully crimped state.

In further examples, the polymeric structure is a polymeric tube. The tube may be bonded on the outer surface of a scaffold, inner surface of a scaffold, or both. In some embodiments, the scaffold is embedded or partially embedded in the tube. The tube may extend from the proximal end of the scaffold to a distal end of the scaffold over the entire surface area of the scaffold including the gaps between struts. In the case of a scaffold with decoupled axial segments, the tube extends over the gaps between axial segments and flexibly binds the segments together. In other examples the tube extends along part of the length of the scaffold.

The tubular structure may be applied and bonded to the scaffold when the scaffold is in an as-cut or expanded configuration, as described above for the elongate elements. The tubular structure may be applied and bonded to the scaffold at a diameter between as-cut (De) or expanded configuration (Dc), as described for the elongate elements.

In some examples the polymeric tube is formed from fibers. For example, the tube can be a woven fibrous mesh with a uniform pattern, such as a helically wound fibers mesh. Alternatively, a fibrous tube may be a plurality of disordered fibers. The fiber tube is permeable and allows passage of cells and blood components, as described above.

In other examples the polymeric tube is a thin-walled tubular film that includes holes through the tube wall to allow passage of cells and blood components, as described above. Except for the through holes, the wall of the polymeric tube may be nonporous. The holes may be distributed throughout the surface or tube. In particular, when the tubular film is placed over a scaffold, there are holes in the portion of the walls of the tube that are over the gaps. The holes may have a width or diameter of less 30 microns, 30 to 50 microns, 50 to 100 microns, 100 to 200 microns, 200 to 300 microns, or greater than 500 microns.

The thickness of the walls of the polymeric tube may be thinner than the width of the struts of the scaffold. For example, the thickness of the polymeric tube may be less than 10%, 10 to 25%, 25 to 50%, 50 to 75%, or 75 to 100% of the thickness of the struts of the scaffold. The polymer tube wall thickness may be less than 20 microns, 20 to 50 microns, 50 to 70 microns, 70 to 100 microns, 100 to 150 microns, or greater than 150 microns.

In some examples the fiber and film tubes do not apply an inward radial force to a scaffold when the stent is in a crimped or as-cut configuration. In some examples, neither the fibrous mesh tube nor the film tube applies an inward radial force to a scaffold when the stent is in a crimped or an as-cut configuration.

The fibers of the fibrous tube and the polymeric film can also be partially bonded to the sidewalls of the scaffold struts. The fibrous tube and polymeric film can also flex inward and outward beyond the inner or outer surface of the struts or into the gap between the struts. In further examples , the scaffold can also be embedded or partially embedded in the fibrous tube.

FIG. 14 depicts an axial projection of a tube 500 of helically wound fiber mesh including two sets of helically wound fibers 504 and 506. Tube 500 has a cylindrical axis A-A. Coordinate system 502 shows the relative orientation with respect to axis A-A. Fibers 504 have a relative orientation greater than 90° and fibers 506 have a relative orientation less than 90°.

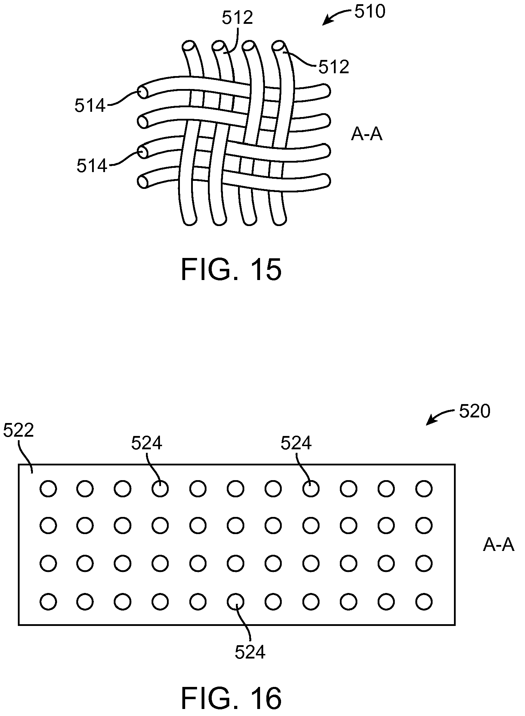

FIG. 15 depicts an alternative fibrous polymer mesh 510 of a fibrous tube. The orientation of the fibers is shown with respect the axis A-A of the fibrous tube made from mesh 510. Mesh 510 includes fibers 512 oriented circumferentially or 90 degrees to axis A-A. Fibers 512 are woven with fibers 514 which are oriented parallel to axis A-A.

FIG. 16 depicts an axial projection of a polymeric film tube 520 having a wall 522. Tube 520 has a plurality of holes 524 between the inner and outer surface of wall 522. Tube 520 has a cylindrical axis A-A.

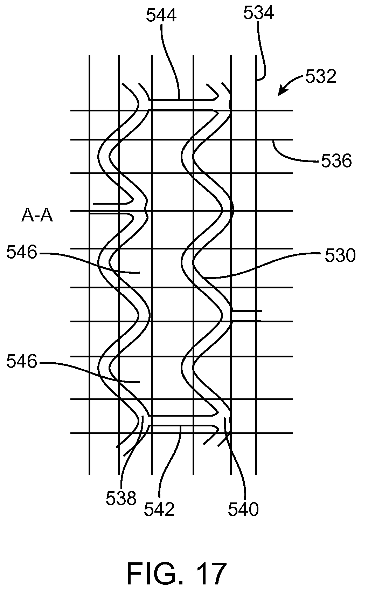

FIG. 17 depicts a portion 530 of a scaffold with a fibrous mesh tube 532 over scaffold 530. Fibrous mesh 532 tube is composed of fibers 534 (parallel to axis A-A of scaffold 530) and fibers 536 that are perpendicular to axis A-A. Fibers 534 and 536 are bonded to ring struts 538 and 540 and extend across the gap defined by ring struts 534, 536 and link struts 542, 544. Pores or gaps in the fibrous mesh formed by the fibers allow permeation of blood components and other cellular material through the fibrous mesh and also allow endothelialization of the scaffold.

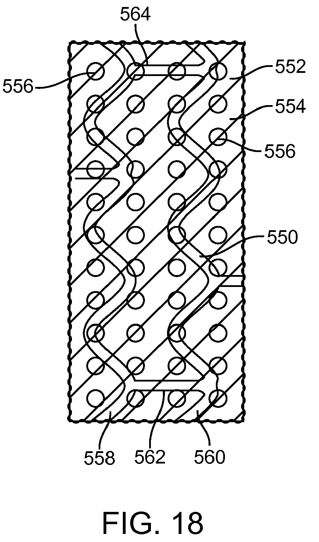

FIG. 18 depicts a portion 550 of a scaffold with a tubular film 552 over scaffold 550. Polymer film tube 552 is composed of polymer film layer 554 (shown in translucent shading). Film layer 554 is bonded to ring struts 558 and 560 and extends across the gap defined by ring struts 558, 560 and link struts 562 and 564. Polymer film layer 554 has holes 556 that allow permeation of blood components and other cellular material through the film layer 554 and also allow endothelialization of the scaffold.

A polymeric tubular structure such as the fibrous tube or polymeric film may be applied and bonded to a scaffold in various ways. The polymeric structure may be applied over an outer surface of a scaffold by disposing the tube over the stent and applying inward radial pressure on the polymer structure and/or outward radial pressure on the scaffold, for example with a crimping device. The scaffold may be mounted over a tubular mandrel having the same or slightly small diameter than the inner diameter of the scaffold. The polymeric structure may be bonded to the scaffold surface using solvent bonding or with an adhesive. A solvent or adhesive can be applied to the scaffold, structure, or both prior to applying pressure to the polymeric structure.

Prior to applying pressure, the polymeric structure may have an inner diameter the same as the outer diameter of the scaffold. The inner diameter of the polymer tube may also be slightly smaller (e.g., up to 1%, 1-2%, 2-5%) or slightly larger (e.g., up to 1%, 1-2%, 2-5%) than the outer diameter of the scaffold. In some examples, when the diameter of the polymeric tube is larger, heat may be applied to the polymeric tube to heat shrink the polymeric tube over the scaffold.

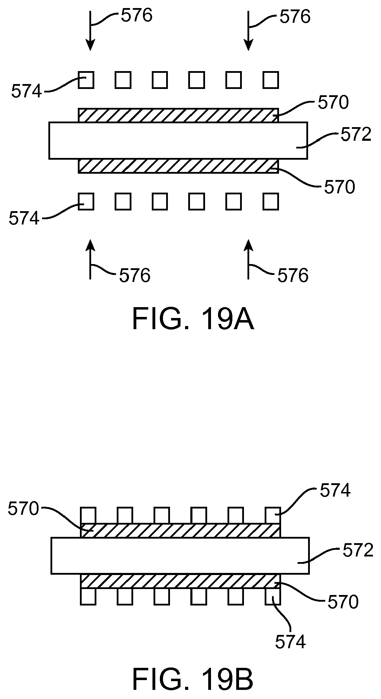

The polymeric tube can be applied and bonded to an inner surface of the scaffold by crimping the scaffold over the polymeric tube. The polymeric structure is bonded using an adhesive or with solvent bonding. FIG. 19A depicts an axial projection of a polymeric tube 570 positioned over a tubular mandrel 572. A scaffold is position over the polymeric tube 570 and mandrel 571. Struts 574 of depicted over polymeric tube 570. As shown, the scaffold has a larger diameter than the polymeric tube 570. Inward radial pressure is applied to the scaffold, as shown by arrows 576, to compress the scaffold against the outer surface of polymeric tube 570, which is in term pressed against the outer surface of mandrel 572. FIG. 19B shows struts 574 of the scaffold pressed against the outer surface of polymeric tube 570.

Another method of applying and bonding a polymeric structure to an inner surface of a scaffold includes disposing a polymeric tube with no gaps or holes in its walls within a scaffold and then disposing the combination within a tubular mold. The tubular mold can have an inner diameter equal or greater than the outer diameter of the scaffold. The polymer tube is then radially expanded by applying pressure to an inside surface of the polymeric tube. This causes the outside surface of the polymeric tube to be pressed against and bonded to the inside surface of the scaffold. The polymeric tube and scaffold may be bonded by an adhesive or by solvent bonding. The polymeric tube can be expanded by inflating a delivery balloon disposed therein. Alternatively, polymeric tube can be expanded by blow molding. The pressure inside the mold can be increased and the expansion can be facilitated by heating the polymeric structure.

In further examples, a polymer structure can include a polymer layer disposed within the gaps of the scaffold and bonded to the side walls that define the gap. In some embodiments, the polymeric layer is also on the inner surface, out surface or both of the scaffold. In other examples the inner and outer surface of the scaffold may be free or partially free of the polymer of the layer. There may additionally be holes in the layer to allow for permeation of blood components and cellular material through the layer.

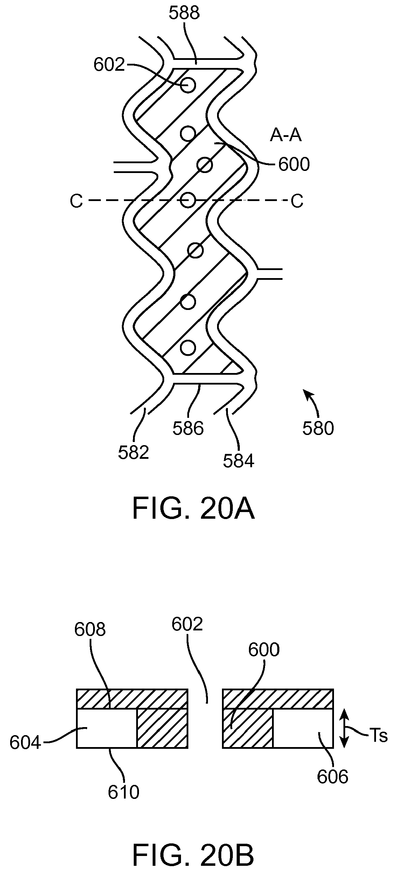

FIG. 20A depicts a section 580 of a scaffold defined by rings 582, 584, link strut 586, and link strut 588. Section 580 includes a polymeric layer 600 disposed within the gap defined ring strut 582, ring strut 584, link strut 586, and link strut 588. Polymeric layer 600 includes through holes 602. FIG. 20B depicts a cross sectional side view across line C-C showing struts 604 and 606 of rings 582 and 584, respectively. Layer 600 is disposed within the gap in the scaffold and also on the outer surface 608 of the scaffold. The layer may also be disposed on an inner surface 610 of the scaffold. Layer 600 can also have a thickness less than the thickness, Ts, of the struts within the gap. The layer may also have an inward or outward flex.

A polymeric layer within the gaps may be formed for example by disposing a scaffold about a tubular mandrel loosely or tightly. A polymer solution may then be applied to the scaffold and within the gaps followed by solvent removal to leave a layer of polymer within the gaps in contact with the side walls of the struts. The application and solvent removal steps can be repeated one or more times to achieve a layer of desired thickness.

In further examples, a scaffold can be composed of axial segments that are connected by flexible links. Such links may be connected to the side walls of struts of adjacent rings of adjacent axial segments. The links connecting adjacent rings of each axial segment may be straight and axially aligned. These segment links may be rigid in the axial direction and be incapable of allowing relative axial movement of rings that the links connect.

The flexible links can be of various shapes such that the axial force contribution to or between the ring segments is minimized or reduced, while maintaining the scaffold in one piece. The flexible link can have curvature to allow bending of the links which allows relative axial movement of adjacent axial segments. One end of a flexible link can be attached at or connected to one circumferential position of an adjacent ring of one axial segment and the other end of the flexible link can be attached at or connected to an adjacent ring of another axial segment at a different circumferential position.

The flexible links may be connected to the rings from a peak or valley of one adjacent ring to a peak or valley of the other adjacent ring. A flexible link may be connected to a ring where a link of the axial segment also meets the ring. In this case, the axial force would be distributed to a greater degree to the other rings of the axial segment, thus reducing the force contribution to ring bending. Alternatively, the flexible ring may be connected to a ring where there is no link of the axial segment.

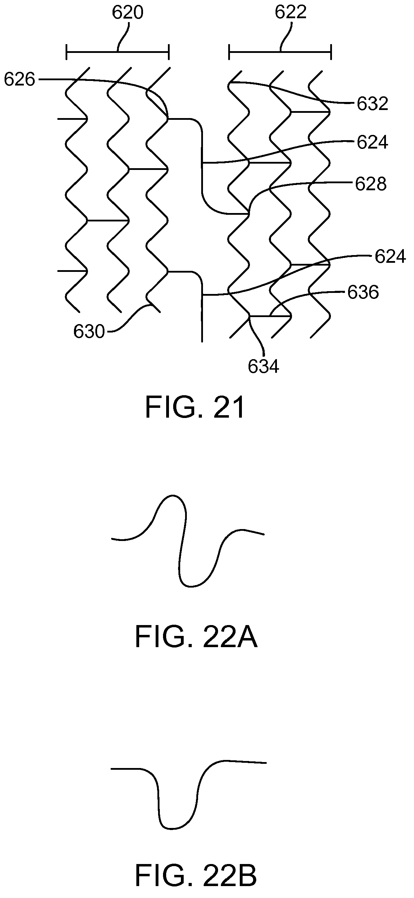

FIG. 21 depicts two adjacent axial segments 620 and 622 of a scaffold. Each axial segment is composed of three rings connected by link struts, with rings 630 and 632 being adjacent rings. A "Z" shaped flexible link 624 connects ring 630 to ring 632. Flexible link 624 is connected to ring 630 at a peak 626 of ring 630. Flexible link 624 is connected to ring 632 at a valley 628 of ring 632. Alternatively, flexible link 624 may be connected to ring 632 at valley 634 where a link 636 is also connected to ring 632.

Other shapes of flexible links can be used to connect the adjacent rings. FIG. 22A depicts an "S" shaped flexible link. FIG. 22B depicts a single loop shaped flexible link.

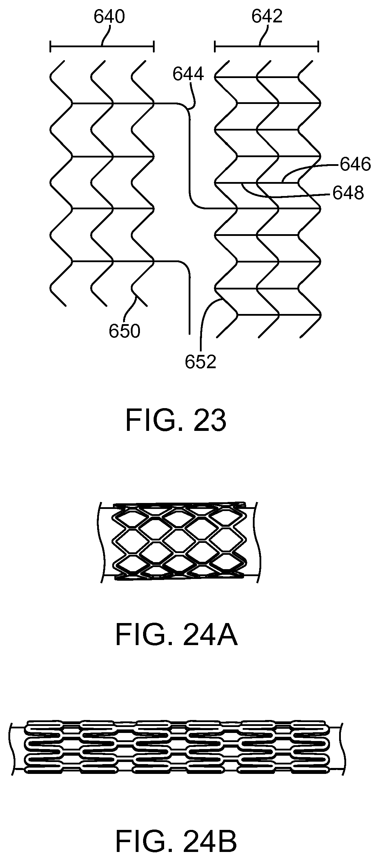

Additionally, links within the axial segments may be arranged to produce more rigid axial segments of rings. This provides a more stable structure that is less affected by axial forces. The rigidity of the axial rings is increased by a greater number of links between rings. FIG. 23 depicts two adjacent axial segments 640 and 642 of a scaffold. Each segment is composed of three rings connected by link struts, with rings 650 and 652 being adjacent rings of adjacent axial segments. Each peak and valley of adjacent rings of each axial segment is connected by link struts, for example, link struts 646 and 648 connect valleys of adjacent rings of axial segment 642.

The scaffold of the present invention can be made from variety of biodegradable polymers including, but not limited to, poly(L-lactide) (PLLA), polymandelide (PM), poly(DL-lactide) (PDLLA), polyglycolide (PGA), polycaprolactone (PCL), poly(trimethylene carbonate) (PTMC), polydioxanone (PDO), poly(4-hydroxy butyrate) (PHB), and poly(butylene succinate) (PBS). The scaffold can also be made from random and block copolymers of the above polymers, in particular, poly(L-lactide-co-glycolide) (PLGA) and poly(L-Lactide-co-caprolactone) PLGA-PCL. The scaffold can also be made of a physical blending of the above polymers. The scaffold can be made from PLGA including any molar ratio of L-lactide (LLA) to glycolide (GA). In particular, the stent can be made from PLGA with a molar ratio of (LA:GA) including 85:15 (or a range of 82:18 to 88:12), 95:5 (or a range of 93:7 to 97:3), or commercially available PLGA products identified as having these molar ratios. High strength, semicrystalline polymers with a Tg above body temperature include PLLA, PGA, and PLGA.

"Radial strength" is the ability of a stent to resist radial compressive forces, relates to a stent's radial yield strength and radial stiffness around a circumferential direction of the stent. A stent's "radial yield strength" or "radial strength" (for purposes of this application) may be understood as the compressive loading, which if exceeded, creates a yield stress condition resulting in the stent diameter not returning to its unloaded diameter, i.e., there is irrecoverable deformation of the stent. When the radial yield strength is exceeded the stent is expected to yield more severely as only minimal additional force is required to cause major deformation. "Stress" refers to force per unit area, as in the force acting through a small area within a plane. Stress can be divided into components, normal and parallel to the plane, called normal stress and shear stress, respectively. Tensile stress, for example, is a normal component of stress applied that leads to expansion (increase in length). In addition, compressive stress is a normal component of stress applied to materials resulting in their compaction (decrease in length). Stress may result in deformation of a material, which refers to a change in length. "Expansion" or "compression" may be defined as the increase or decrease in length of a sample of material when the sample is subjected to stress.

As used herein, the terms "axial" and "longitudinal" are used interchangeably and refer to a direction, orientation, or line that is parallel or substantially parallel to the central axis of a stent or the central axis of a tubular construct. The term "circumferential" refers to the direction along a circumference of the stent or tubular construct. The term "radial" refers to a direction, orientation, or line that is perpendicular or substantially perpendicular to the central axis of the stent or the central axis of a tubular construct and is sometimes used to describe a circumferential property, i.e radial strength.

"Strain" refers to the amount of expansion or compression that occurs in a material at a given stress or load. Strain may be expressed as a fraction or percentage of the original length, i.e., the change in length divided by the original length. Strain, therefore, is positive for expansion and negative for compression.

"Strength" refers to the maximum stress along an axis which a material will withstand prior to plastic deformation and then fracture. The ultimate strength is calculated from the maximum load applied during the test divided by the original cross-sectional area.

"Modulus" may be defined as the ratio of a component of stress or force per unit area applied to a material divided by the strain along an axis of applied force that result from the applied force. For example, a material has both a tensile and a compressive modulus.

The underlying structure or substrate of an implantable medical device, such as a stent can be completely or at least in part made from a biodegradable polymer or combination of biodegradable polymers, a biostable polymer or combination of biostable polymers, or a combination of biodegradable and biostable polymers. Additionally, a polymer-based coating for a surface of a device can be a biodegradable polymer or combination of biodegradable polymers, a biostable polymer or combination of biostable polymers, or a combination of biodegradable and biostable polymers.

### Examples of PLLA segmented scaffolds

FIG. 24A depicts an axial scaffold segment with a pattern similar to that of FIG. 6A in an as cut configuration. The short link struts are 0.010 in. FIG. 24B depicts the axial scaffold segment in a crimped stated. The segment was found to crimp evenly.

The crimping process included three steps:
1. Place segments on length of balloon at >2mm spacing
2. Crimp to near fully crimped
3. Set segment spacing at 1mm and then fully crimp.

An alternative to this process is to pre-crimp all segments to near fully crimped without a balloon. Then feed onto balloon at selected spacing between segments and then fully crimp. The friction between balloon and scaffold set by the pre-crimp may hold the segments in place during setting of the spacing and the final crimp stages.

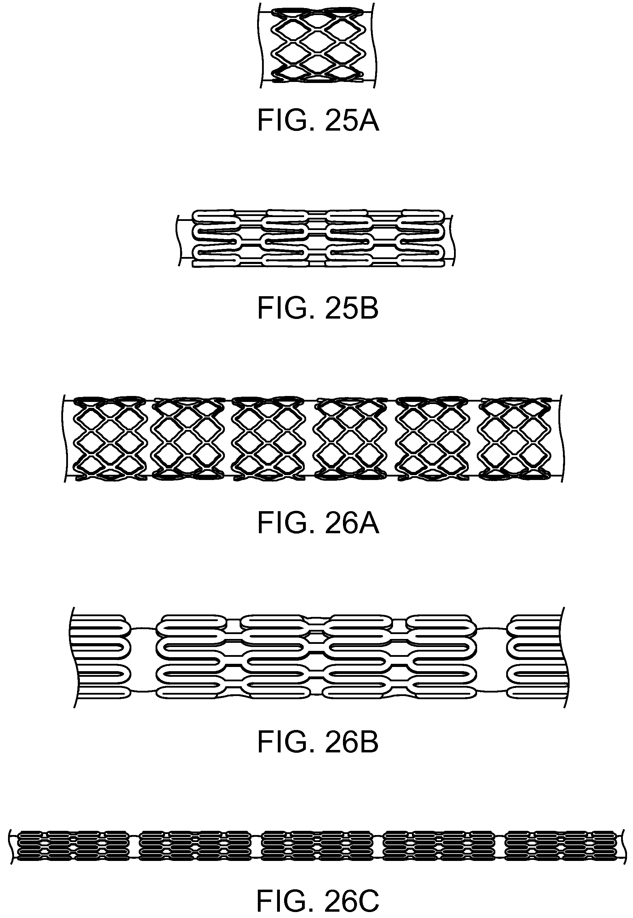

FIG. 25A depicts an axial scaffold segment in an as cut configuration with a pattern similar to that of FIG. 6A which additionally includes keyhole features as depicted in FIG. 6D. The short link struts are 0.010 in. FIG. 25B depicts the scaffold segment in a crimped state. The keyholes make rings wider and crimp recoil was observed. The ring width can be reduced to that in FIG. 24B and the key hole can be tapered for less strut interference when crimped.

FIG. 26A depicts six scaffold segments disposed over a balloon prior to crimping. FIG. 26B depicts a close-up of one segment after crimping illustrating the balloon pillowing between scaffold segments. This aids in maintaining segment spacing during delivery and balloon deployment. FIG. 26C depicts five scaffold segments after crimping.

FIG. 27 depicts an exemplary axial scaffold segment with a pattern as shown in FIG. 6C. The axial segments include a tapered key hole and a link length of 0.02 in. A longer link was used to avoid ring to ring interference.

Scaffold segments were mounted and crimped onto a balloon and deployed to a diameter of about 6.77 mm. The diameter was monitored over a period of 30 min.

The recoil from the deployed diameter was between 5.5 and 6% 30 min after deployment. In comparison, recoil of a non-segmented PLLA scaffold such as that shown in FIG. 1 and disclosed in US20110190872 was about 8%.

The radial strength and radial stiffness of the segmented scaffold are shown in FIGs. 28A and 28B, respectively. Also, shown is the radial strength and stiffness of the non-segmented scaffold.

FIG. 29 depicts the crush recovery of the segmented scaffold and segmented scaffold after 50% crush.

While particular embodiments of the present invention have been shown and described, it will be obvious to those skilled in the art that changes and modifications can be made without departing from this invention in its broader aspects. Therefore, the appended claims are to encompass within their scope all such changes and modifications as fall within the scope of this invention.

## Claims

1. A scaffold (300), comprising:
two or more radially expandable axial scaffold segments (351, 352, 353, 354, 355, 356) arranged end to end,
wherein each axial segment includes 2 or more cylindrical rings (372, 374) composed of undulating ring struts forming crests (377, 379) and troughs (378, 380), and
wherein adjacent cylindrical rings in the same axial segment comprise one or more link struts (376) connecting the adjacent cylindrical rings forming adjacent ring pairs, and
**characterized in that**
the scaffold is made from a polymeric tube or polymeric sheet, and **in that** for each adjacent ring pair the troughs (378, 380) of one of
the rings are aligned with the crests (377, 379) of the other ring and the troughs of the other ring are aligned with the crests of the one ring, such that
a trough of the one ring is separated by a first distance from a crest of the other ring,
a crest of the one ring is separated by a second distance from a trough of the other ring,
the first distance is less than the second distance, and
only every third aligned crest of the other ring and trough of the one ring is connected by a link strut.

2. The scaffold of claim 1, wherein the axial segments are disposed over a delivery balloon (350).

3. The scaffold of claim 1, wherein the segments are deployable within a blood vessel.

4. The scaffold of claim 1, wherein the ends of adjacent axial segments are spaced apart at least a distance between rings in the axial segments.

5. The scaffold of claim 1, wherein the ends of adjacent axial segments are spaced apart less than a two times a length of the linking struts.

6. The scaffold of claim 1, wherein the axial segments are crimped to a balloon (350).

## Patentansprüche

1. Ein Gerüst (300), umfassend:
zwei oder mehrere radial ausdehnbare axiale Gerüstabschnitte (351, 352, 353, 354, 355, 356), die mit deren Enden aneinander angeordnetsind,
wobei jeder axiale Abschnitt zwei oder mehrere zylindrische Ringe (372, 374) umfasst, die aus wellenförmigen Ringstreben bestehen, welche Wellenberge (377, 379) und Wellentäler (378, 380) bilden, und
wobei angrenzende zylindrische Ringe im gleichen axialen Abschnitt eine oder mehrere Verbindungsstreben (376) umfassen, welche die angrenzenden zylindrischen Ringe verbinden und dadurch angrenzende Ringpaare bilden, und
**dadurch gekennzeichnet, dass**
das Gerüst aus einem polymeren Rohr oder einer polymeren Schicht besteht und dadurch, dass die Täler (378, 380) eines
der Ringe für jedes Paar angrenzender Ringe mit den Bergen (377, 379) des anderen Ringes fluchten und die Täler des anderen Ringes mit den Bergen des einen Ringes fluchten, so dass
ein Tal des einen Ringes durch einen ersten Abstand von einem Berg des anderen Ringes getrennt ist,
ein Berg des einen Ringes durch einen zweiten Abstand von einem Tal des anderen Ringes getrennt ist,
der erste Abstand geringer als der zweite Abstand ist, und
nur jeder dritte gefluchtete Berg des anderen Ringes und Tal des einen Ringes durch eine Verbindungsstrebe verbunden ist.

2. Das Gerüst des Anspruchs 1, wobei die axialen Abschnitte über einen abgebenden Katheterballon (350) angeordnet sind.

3. Das Gerüst des Anspruchs 1, wobei die Abschnitte innerhalb eines Blutgefäßes entfaltet werden können.

4. Das Gerüst des Anspruchs 1, wobei die Enden von angrenzenden axialen Abschnitten durch mindestens einen zwischen Ringen in den axialen Abschnitten existierenden Abstand voneinander getrennt sind.

5. Das Gerüst des Anspruchs 1, wobei die Enden von angrenzenden axialen Abschnitten weniger als zweimal eine Länge der Verbindungsstreben voneinander getrennt sind.

6. Das Gerüst des Anspruchs 1, wobei die axialen Abschnitte gecrimpt sind und dabei einen Ballon (350) bilden.

## Revendications

1. Un échafaudage (300), comprenant :
deux ou plusieurs segments d'échafaudage axiaux radialement expansibles (351, 352, 353, 354, 355, 356) disposés bout à bout l'un par rapport à l'autre,
dans lequel chaque segment axial inclut deux ou plusieurs anneaux cylindriques (372, 374) composés d'entretoises d'anneau ondulées formant des crêtes (377, 379) et des creux (378, 380), et
dans lequel des anneaux cylindriques adjacents dans le même segment axial comprennent une ou plusieurs entretoises de liaison (376) qui relient les anneaux cylindriques adjacents formant des paires d'anneaux adjacents, et
**caractérisé en ce que**
l'échafaudage est fait à partir d'un tube polymère ou d'une feuille polymère, et **en ce que** pour chaque paire d'anneaux adjacents les creux (378, 380) d'un des
anneaux sont alignés avec les crêtes (377, 379) de l'autre anneau et les creux de l'autre anneau sont alignés avec les crêtes de l'un anneau, de façon que
un creux de l'un anneau est séparé d'une crête de l'autre anneau par une première distance,
une crête de l'un anneau est séparée d'un creux de l'autre anneau par une deuxième distance,
la première distance est inférieure à la deuxième distance, et
seulement chaque troisième crête alignée de l'autre anneau et creux de l'un anneau est relié(e) moyennant une entretoise de liaison.

2. L'échafaudage de la revendication 1, dans lequel les segments axiaux sont disposés au-dessus d'un ballonnet d'administration (350).

3. L'échafaudage de la revendication 1, dans lequel les segments peuvent être déployés dans un vaisseau sanguin.

4. L'échafaudage de la revendication 1, dans lequel les bouts de segments axiaux adjacents sont séparés au moins une distance existante entre des anneaux dans les segments axiaux.

5. L'échafaudage de la revendication 1, dans lequel les bouts de segments axiaux adjacents sont séparés moins de deux fois une longueur des entretoises de liaison.

6. L'échafaudage de la revendication 1, dans lequel les segments axiaux sont sertis formant un ballonnet (350).
